# EUROPÄISCHE PATENTANMELDUNG

(11) **EP 3 366 344 A2**
(43) Veröffentlichungstag der Anmeldung: **29.08.2018**
(21) Anmeldenummer: 18156804.9
(22) Anmeldetag: 14.02.2018
(51) Int. Cl.: A61M 35/00, B05B 16/00

(54) **VORRICHTUNG ZUM AUFTRAGEN VON SONNENSCHUTZMITTELN UND BEHÄLTER ZUR VERWENDUNG MIT EINER SOLCHEN**

(30) Priorität: 22.02.2017 DE 102017103671
(71) Anmelder: Julie Soley GmbH, 67433 Neustadt an der Weinstrasse (DE)
(72) Erfinder: Szkutnik, Daniel, 68165 Mannheim (DE); Conrad, Carsten, 67433 Neustadt/Weinstraße (DE); Englert, Julia, 74889 Sinsheim (DE); Rößler, Patrick, 68199 Mannheim (DE)
(74) Vertreter: Geitz Truckenmüller Lucht Christ

(57) **Zusammenfassung**

Um einen vollständigen Sonnenschutz auf der Haut aufzutragen ist es bereits bekannt, anstelle eines manuellen Einreibens des Sonnenschutzmittels eine Sonnenschutzdusche zu benutzen, welche das Sonnenschutzmittel über eine Düsenanordnung auf die Haut sprüht. Allerdings benötigen unterschiedliche Hauttypen auch unterschiedliche Sonnenschutzmittel, so dass im Stand der Technik auch Weiterentwicklungen bekannt sind, die mehrere verschiedene Sonnenschutzmittel ausbringen können.

Hiervon ausgehend erhöht die Erfindung den Grad der Individualisierung ganz erheblich, indem sie eine Duscheinheit mit einer Behälteraufnahme vorsieht, in die ein individueller Behälter eingesetzt werden kann, deren Inhalt dann im Inneren der Duscheinheit versprüht wird. Zur Gewährleistung der Funktion erfolgt vor der Inbetriebnahme eine Überprüfung des eingelegten Behälters hinsichtlich dessen Kompatibilität.

## Beschreibung

Die vorliegende Erfindung betrifft eine Vorrichtung zum Auftragen von Sonnenschutzmitteln auf die menschliche Haut, umfassend eine Umfangswandung mit einer Düsenanordnung zum Ausbringen des Sonnenschutzmittels in einen von der Umfangswandung umgebenen Raumbereich, wobei die Düsenanordnung über eine Druckleitung mit Mitteln zum Vorhalten von Sonnenschutzmittel verbunden ist, sowie einen Behälter zur Verwendung mit einer solchen Vorrichtung.

Eine Duscheinheit zum Auftragen von Sonnenschutzmittel der vorgenannten Art ist bereits aus der DE 299 03 703 U1 vorbekannt. Sie weist eine Duschkabine mit einem Innenraum, in dem das Sonnenschutzmittel versprüht wird, und einen Außenraum, der zum Entkleiden und Aufbewahren von persönlichen Gegenständen während des Duschvorgangs vorgesehen ist, auf. Im Boden der Duscheinheit ist hierbei ein Behälter mit Sonnenschutzmittel vorgehalten, der über eine Saugpumpe und eine Hebevorrichtung mit einer Düsenanordnung verbunden ist.

Betritt ein Benutzer den Innenraum und löst den Duschvorgang durch Betätigung eines Münzschalters und verschließen einer Innenraum-Tür aus, so wird das Sonnenschutzmittel aus dem Behälter gesaugt und über die Düsenanordnung in den Innenraum gesprüht.

Hierbei verfahren die Düsenelemente der Düsenanordnung zwischen zwei Endlagenschaltern auf und ab, so dass eine räumlich begrenzte Düsenanordnung eine vollständige Benetzung des Körpers erlaubt.

Eine solche Duscheinheit funktioniert ohne Weiteres für Benutzer einer bestimmten Größe, da die Lage der Endschalter die oberste Sprühhöhe vorgeben. Es kann dem Sprühen auf das Kopfhaar und in das Gesicht allenfalls durch eine Verdeckung dieser Bereiche begegnet werden.

Die US 2007/0107121 A1 nimmt sich dieser Problematik an und tastet den Körper des im Innenraum stehenden Benutzers hinsichtlich seiner Größe ab und verfährt die Düsenanordnung entsprechend, so dass verschiedene Zonen identifiziert und unterschiedlich mit Sonnenschutzmittel beaufschlagt werden können. Hierdurch kann ferner der Sprühdruck für die verschiedenen Körperbereiche angepasst werden und bedarfsweise ist es möglich, den Kopfbereich auszusparen. Dies spart zum Einen Sonnenschutzmittel, das nicht unnötig auf ungewünschte Körperstellen aufgebracht wird, zum anderen verbessert dieses Vorgehen das Erlebnis für den Benutzer. Ein abwärts gerichteter Luftstrom sorgt hierbei zusätzlich dafür, dass das Sonnenschutzmittel nicht aufwärts und in Richtung des Gesichts verwirbelt werden kann, sondern vielmehr stets nach unten und damit vom Gesicht des Benutzers weg gedrückt wird.

Mit einer derartigen Anpassung wird zwar auf die Größe und die Proportionen des jeweiligen Benutzers eingegangen, jedoch nicht auf dessen Hautbild. Über diesen Aspekt wird jedoch beispielsweise in der DE 20 2009 015 578 U1 nachgedacht. Dort erfolgt vor dem Auftragen des Sonnenschutzmittels eine selbsttätige Erkennung des Hauttyps und es wird das Alter und das Geschlecht des Benutzers abgefragt. Unter Berücksichtigung von Geodaten, Kalenderdaten und Wetterdaten wird ein Sonnenschutzmittel ausgewählt oder zusammengemischt und aufgetragen.

Dies scheint zunächst eine Möglichkeit darzustellen, einen optimalen Schutz für die Haut zu erhalten, wobei Individuelle Wünsche des Benutzers nicht ausreichend berücksichtigt werden. Eine Auswahl hinsichtlich der Marke, Qualität, gegebenenfalls des Geruchs, kann hier nicht getroffen werden. Zudem funktioniert die Einrichtung nur so lange, wie die zahlreichen vorzuhaltenden Komponenten regelmäßig nachgefüllt werden.

Das gleiche Problem ergibt sich auch für den Gegenstand der US 6 918 897 B2, bei dem zwar eine Auswahl des aufzutragenden Sonnenschutzmittels durchaus manuell möglich ist, aber die Auswahl nur auf die gerade vorhandenen Mittel beschänkt bleibt, auf deren Füllstand nur das Wartungspersonal einen Einfluss hat.

Es verbleibt jedoch das ungelöste Problem, die Duscheinheit so weit zu individualisieren, dass der Benutzer mit dem von ihm selbst gewünschten Sonnenschutzmittel eingesprüht werden kann.

Gelöst wird diese Aufgabe durch eine Vorrichtung zum Auftragen von Sonnenschutzmitteln auf die menschliche Haut gemäß den Merkmalen des unabhängigen Anspruchs 1. Ferner wird dieses Problem durch einen mit dieser Vorrichtung zu verwendenden Behälter gemäß den Merkmalen des unabhängigen Anspruchs 11 gelöst. Die übrigen Ansprüche stellen sinnvolle Weiterbildungen der Vorrichtung oder des Behälters dar.

Erfindungsgemäß ist vorgesehen, dass eine Vorrichtung zum Auftragen von Sonnenschutzmitteln als Duscheinheit gebildet ist, welche eine Umfangswandung besitzt. Dies ist schon als Sicht- und Spritzschutz geboten, dient aber auch zur Aufnahme einer Düsenanordnung, von der aus das Sonnenschutzmittel in den Raumbereich innerhalb der Umfangswandung eingebracht wird. Die Düsenanordnung ist über eine Druckleitung mit einem Behälter verbunden, in dem das Sonnenschutzmittel vorgehalten wird. Dieser Behälter wird jedoch in eine Behälteraufnahme eingelegt, die Mittel zur Behälterauthentifizierung aufweist, um sicherzustellen, dass nur für den Einsatz mit der erfindungsgemäßen Vorrichtung geeignete Behälter eingesetzt werden. Ferner weist die Behälteraufnahme Mittel zur Behälteröffnung auf, um den Inhalt des Behälters freigeben zu können und das Innere des Behälters mit der Druckleitung zu verbinden. Nach der Erkennung des Behälters wird dieser mit den Mitteln zur Behälteröffnung geöffnet und über einen Ansaugstutzen, der mit den Mitteln zur Behälteröffnung einstückig gebildet sein kann, das im Behälter vorgehaltene Sonnenschutzmittel herausgesaugt. Über die Druckleitung wird das Sonnenschutzmittel bis zu der Düsenanordnung weitergeleitet und über diese in den von der Umfangswandung umgebenen Raumbereich, in dem der Benutzer sich positioniert hat, abgegeben.

Durch die Verwendung einer Behälteraufnahme, welche eine Identifizierung des Behälters ermöglicht und den Inhalt des Behälters in die Druckleitung einspeisen kann, ist es möglich, dass der Benutzer sein Sonnenschutzmittel im Vorfeld in einem passenden Behälter erwirbt und einsetzt. Hierdurch kann zudem eine separate Bezahlung vor Ort in der Dusche entfallen, da beim Erwerb eines berechtigten Behälters auch die Berechtigung zur Nutzung der Vorrichtung mit erworben wird. Ein Vorhalten von Geld, Kreditkarten oder Wertbons kann damit erfindungsgemäß entfallen. Vielmehr kann der Benutzer vorab entscheiden, welches Sonnenschutzmittel welches Herstellers er benutzen möchte und welchen Sonnenschutzfaktor er bevorzugt. Zudem ist die Vorrichtung durch das Mitbringen des Behälters durch den Benutzer stets betriebsbereit, da es hierdurch nie an Betriebsmitteln fehlen kann.

Bei den Mitteln zur Behälterauthentifizierung kann es sich um eine polyedrisch geformte Behältermatritze handeln, welche eine Kavität zur formschlüssigen Aufnahme des vom Benutzer gewählten Behälters mit Sonnenschutzmittel aufweist. Alternativ oder ergänzend können die Mittel zur Behälterauthentifizierung auch elektronische, optische und/oder magnetische Kennzeichnungsmittel umfassen, die auf entsprechende Markierungen des Behälters reagieren und die Benutzung des Behälters nur dann freigeben, wenn die Authentifizierung durch die Mittel zur Behälterauthentifizierung erfolgreich verlaufen ist.

Einerseits soll hierdurch verhindert werden, dass ungeeignete Sonnenschutzmittel, etwa mit zu geringer Viskosität oder problematischem Sprühverhalten, eingesetzt werden, andererseits soll die Benutzung der Duscheinheit an den Erwerb der Behälter gekoppelt werden, so dass eine Benutzung anderer Behälter dieser Intention zuwiderlaufen würde.

Im Rahmen einer elektronischen Authentifizierung kann auch eine Identifizierung eines einzelnen Behälters erfolgen, indem etwa ein in den Behälter integrierter Speicherchip ausgelesen und über eine Datenverbindung mit einer Datenbank abgeglichen wird. Durch eine Entwertung bereits benutzter Behälter in der Datenbank kann auch eine mehrfache Benutzung desselben Behälters, etwa durch eine Neubefüllung, ausgeschlossen werden.

Die Behälteraufnahme kann innerhalb eines Außenraums der Vorrichtung angeordnet sein, so dass mit dem Einlegen des Behälters und dessen Authentifizierung der Vorgang gestartet und eine Außentür des Außenraums daraufhin selbsttätig geschlossen werden kann. Durch das Verschließen einer weiteren Tür zu dem von der Umfangswand umgebenen Raumbereich kann dann der Duschvorgang durch den Benutzer letztendlich gestartet werden.

Mit einigem Vorteil wird nach dem Beginn des Duschvorgangs, zumindest aber nach dem Einlegen eines Behälters in die Behälteraufnahme, dieser Behälter geöffnet und sein Inhalt in einen Zwischenspeicher in der Druckleitung eingesaugt. Dies ermöglicht einen gleichmäßigen Zugriff auf das Sonnenschutzmittel während des Duschvorgangs und begünstigt, dass der gesamte Systemdruck initial für das Entleeren des Behälters zur Verfügung steht.

Ferner kann der Druckleitung ein Reinigungsmittelbehälter zugeschaltet werden, über den eine Selbstreinigung der Vorrichtung über die Düsenanordnung erfolgen kann. Ergänzend oder Alternativ kann auch eine Verbindung mit einer Wasserversorgung hergestellt werden. Nach dem Duschvorgang kann zusätzlich eine Benetzung der Umfangswand und des Bodens erfolgen, indem Reinigungsmittel, Wasser oder eine Mischung daraus über eine am oberen Rand der Umfangswand angeordnete Reinigungsdüsenanordnung ausgeströmt wird und eventuelle Anhaftungen von Schmutz und/oder Sonnenschutzmittel mitnimmt.

Um mit dem Sonnenschutzmittel zu haushalten und die eingangs beschriebenen negativen Effekte einer Besprühung des Gesichts des Benutzers zu vermeiden, können der Umfangswandung zudem Mittel zur Bestimmung der Körpergröße zugeordnet sein. Für eine derartige Lösung gibt es zahlreiche mögliche Ansätze, wobei allerdings Bildaufnahmen zur Auswertung in dem geschlossenen Bereich zu vermeiden wären. Die Verwendung einer oberseitig angeordneten Time of Flight-Kamera würde ein Höhenraster liefern, das hinsichtlich der Körpergröße ausgewertet werden könnte. Auch könnte mit Wellenreflexionen von Kopf und Schultern gearbeitet werden.

Eine bevorzugte Ausgestaltung sieht vor, dass die Mittel zur Bestimmung der Körpergröße wenigstens eine Lichtquelle und mehrere in unterschiedlichen Höhenlagen oder höhenverfahrbar angeordnete Fotorezeptoren gegenüber der Lichtquelle, oder falls mit Reflexionen gearbeitet wird um die Lichtquelle herum, angeordnet sind. Im Falle einer höhenverfahrbaren Einrichtung werden die Fotorezeptoren entlang der Umfangswand so lange abwärts bewegt, bis ein zunächst bestehender Kontakt abbricht. Im Fall einer stationären Einrichtung wird geprüft, an welchen Fotorezeptoren Licht ankommt und ausgehend davon auf die Körpergröße des Benutzers zurückgeschlossen.

Die Düsenanordnung zum Ausbringen des Sonnenschutzmittels besteht aus mehreren Düsenelementen, die mit der Druckleitung verbunden sind. Sie können an vertikal an der Umfangswandung angeordneten Schienen verfahrbar oder stationär und in vertikaler Richtung schwenkbar sein, so dass sie in Abhängigkeit von der ermittelten Körpergröße des Benutzers eingerichtet werden können.

Ferner kann die Umfangswandung eine Fluiddüsenanordnung zugeordnet sein, aus der ein Fluidmantel ausströmen kann um Sprühnebelverwirbelungen zu vermeiden. Aufgrund des Fluidmantels, der im Bereich der Umfangswandung herabfällt, kann somit kein Sonnenschutzmittel in das Gesicht oder an den Hinterkopf des Benutzers gelangen, so dass ein Augenkontakt oder ein Verkleben der Kopf und/oder Barthaare mit dem Sonnenschutzmittel vermieden ist. Der Fluidmantel kann aus einem Gas, vorzugsweise angesaugter Umgebungsluft, aber auch aus einer Flüssigkeit, etwa dünnen Wasserstrahlen oder einem Wassernebel, gebildet sein. Neben dem Schutz vor aufsteigendem Sprühnebel sorgt dieser Effekt auch dafür, dass die Verschmutzung der Umfangswand mit Sonnenschutzmittel allenfalls geringe Ausmaße annehmen kann.

Zur Benutzung der vorbeschriebenen Vorrichtung wird ein Behälter mit Sonnenschutzmittel benötigt, wobei es sich um einen Behälter mit einer langgestreckten, polyedrischen Außenkontur handeln kann. Die Außenkontur soll mit der Behältermatritze der Behälteraufnahme derart zusammenpassen, dass nur Behälter mit der gewählten polyedrischen Form in die Behälteraufnahme passen, so dass keine ungeeigneten Sonnenschutzmittel eingesetzt werden können.

Die langgestreckte Form sorgt hierbei dafür, dass der Behälter nach dem Einlegen leicht ausgesaugt werden kann und das Sonnenschutzmittel möglichst vollständig zur Verfügung steht. Die Größe des Behälters kann zudem in der Länge angepasst werden, um unterschiedliche Portionsgrößen, für Erwachsene und für Kinder, zu ermöglichen.

Da die Vorrichtung in einer bevorzugten Ausführungsform einen Fluidmantel einsetzt, der ein Benetzen des Kopfes und des Gesichts vermeiden soll, kann der Behälter ebenfalls bevorzugtermaßen über eine Trennkante in zwei Behälterteile mit voneinander flüssigkeitsdicht getrennten Teilkammern aufgespalten werden. Der größere Teil wird in diesem Fall in die Behälteraufnahme eingesetzt und über die Düsenanordnung in den von der Umfangswandung umgebenen Raumbereich eingebracht. Der kleinere Teil hingegen verbleibt bei dem Benutzer und kann anschließend zum Einreiben der von der Vorrichtung nicht benetzten Körperteile, also Gesicht, gegebenenfalls Hinterkopf und Fußsohlen, eingesetzt werden.

Um das aufgrund des Einsatzes von Einwegbehältern zu erwartende Abfallvolumen möglichst überschaubar zu halten, wird der Behälter aus einem biologisch abbaubaren Kunststoff hergestellt und kann daher kompostiert werden.

Die vorstehend beschriebene Erfindung wird im Folgenden anhand eines Ausführungsbeispiels näher erläutert.

Es zeigen
- Figur 1: eine erfindungsgemäße Duscheinheit in einer schematischen Perspektivdarstellung von schräg oben,
- Figur 2: eine frontale und schematische Draufsicht auf die abgerollte Umfangswandung der Duscheinheit gemäß Figur 1,
- Figur 3: ein schematisches Funktionsschaubild der Sonnenschutzmittelleitung innerhalb der Duscheinheit gemäß Figur 1, sowie
- Figur 4: einen erfindungsgemäßen Behälter in einer perspektivischen Darstellung von schräg oben.

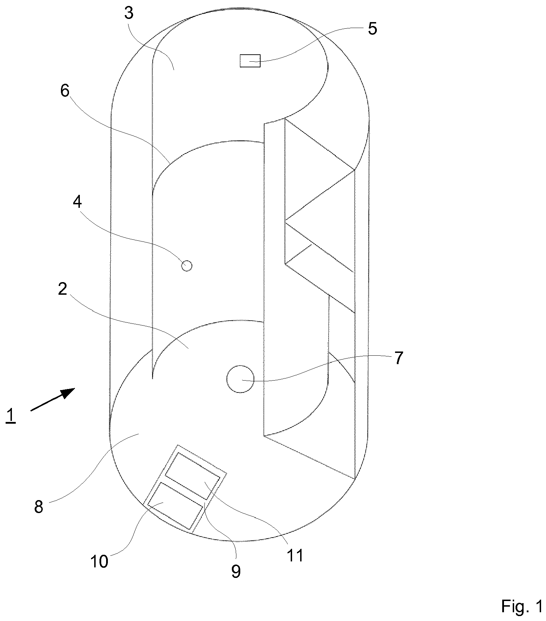

Figur 1 zeigt eine Duscheinheit 1, welche im Wesentlichen aus einem Außenraum 8 sowie einem darin eingesetzten Innenraum 2 besteht. Beide Teilräume sind jeweils über eine nicht gezeichnete Tür verschließbar. Die Duscheinheit 1 bildet eine Kabine, in der sich ein Benutzer in kurzer Zeit vollständig mit einem selbst ausgewählten Sonnenschutzmittel einsprühen lassen kann. Nach dem Betreten des Außenraums 8 durch einen Benutzer schließt die Tür des Außenraums selbsttätig und lässt sich auch nur von innen wieder öffnen. Regale und Haken sowie ein Fach für Wertgegenstände bieten Platz für die Ablage der Kleidung oder Badekleidung. Über ein Benutzerinterface kann der Benutzer Einstellungen vornehmen und über eine Behälteraufnahme 18 einen zuvor erworbenen Behälter 28 mit der Duscheinheit 1 verbinden. Auch dient das Benutzerinterface dazu, den Benutzer möglichst intuitiv an die Interaktion mit der Kabine heranzuführen und ihm die nächsten Schritte wie ausziehen, in den Innenraum gehen und so weiter aufzuzeigen. In einer alternativen Ausgestaltung schließt die Tür des Außenraums nur nach dem Einlegen und Authentifizieren eines geeigneten Behälters 28.

Nach dem Ablegen seiner Kleidung betritt der Benutzer einen Innenraum 2, welcher von einer Umfangswandung 3 begrenzt wird. Lichtschranken 4 stellen fest, dass der Innenraum 2 belegt ist und manuell oder auf Knopfdruck wird eine Tür zum Innenraum 2 geschlossen, um ein Austreten von Sonnenschutzmittel aus dem Innenraum 2 in den Außenraum 8 zu verhindern. Mithilfe von Mitteln zur Bestimmung der Körpergröße 5 wird sodann die Körpergröße des Benutzers ermittelt und die im späteren Verlauf beschriebene Düsenanordnung 14 eingestellt, so dass sie sich lediglich auf Körperbereiche unterhalb des Halses des Benutzers richtet.

Über die Düsenanordnung 14 an der Umfangswand 3 wird dann aus dem Behälter 28 ausgesaugtes Sonnenschutzmittel in den Innenraum 2 eingesprüht und die Haut des Benutzers dadurch benetzt. Über eine Fluiddüsenanordnung 6 tritt ein Fluidmantel, etwa ein Luftmantel, aus und fällt nach unten, so dass eventuell entstehender Sprühnebel aus Sonnenschutzmittel nicht aufsteigen kann.

Während oder nach jedem Duschvorgang wird über die Düsenanordnung 14 Reinigungsmittel in dem Innenraum 2 versprüht, um diesen zu reinigen. Ergänzend wird Reinigungsmittel auch über eine Reinigungsdüsenanordnung 12 ausgeströmt, das die Umfangswandung 3 benetzt und reinigt. Die Umfangswandung 3 hat eine Nanobeschichtung, so dass Schmutz, das Sonnenschutzmittel, Reinigungsmittel und Wasser von ihr abperlen. Das Reinigungsmittel wird über einen Abfluss 7 im Boden des Innenraums 2 abgeführt, über eine Bodenklappe 10 im Außenraum 8 kann ein Reinigungsmittelbehälter 11 sowie ein mit dem Abfluss 7 verbundener Fettabscheider zugegriffen werden.

Die Duscheinheit 1 ist in der Grundform an einen Zylinder angelehnt. Die von ihr gebildete Kabine ist nach oben offen ausgeführt, so dass sie eine ausreichende Helligkeit und eine gute Luftzirkulation ermöglicht.

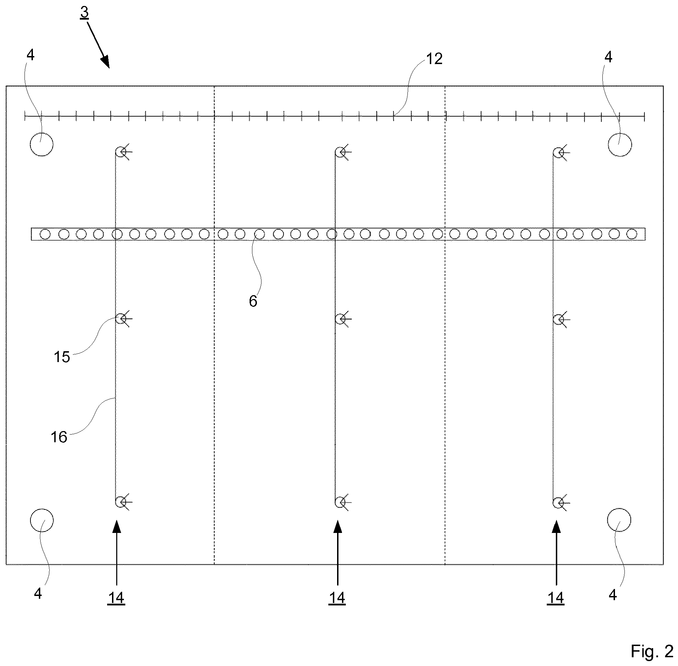

Figur 2 zeigt eine Draufsicht auf eine abgerollte Umfangswandung 3, die mit mehr Details gezeichnet ist als zuvor in Figur 1. An der Umfangswandung 3 sind mehrere Schienen 16 befestigt, an denen sich Düsenelemente 15 der Düsenanordnung 14 befinden. Im vorliegenden Beispiel sind jeweils drei Düsenelemente 15 einzeln oder im Verbund höhenverstellbar und können entlang ihrer jeweiligen Schiene 16 verschoben werden, was in Abhängigkeit von der eingangs gemessenen Körpergröße des Benutzers geschieht. Die Düsenelemente 15 sind über eine Druckleitung 25 letztlich mit dem vom Benutzer mitgebrachten und in die Behälteraufnahme 18 eingelegten Behälter 28 verbunden und bringen das darin eingangs enthaltene Sonnenschutzmittel als Sprühnebel in den Innenraum 2 ein.

Ebenfalls höhenverstellbar ist an der Umfangswandung die Fluiddüsenanordnung 6 zum Ausbringen von Luft zur Bildung eines den Aufstieg von Sprühnebel vermeidenden Fluidmantels angeordnet. An der Umfangswandung 3 im oberen Bereich angeordnet ist eine Reinigungsdüsenanordnung 12, über welche Reinigungsmittel, Wasser, oder eine Mischung daraus, nach dem Duschvorgang über die Umfangswandung ausgeströmt wird.

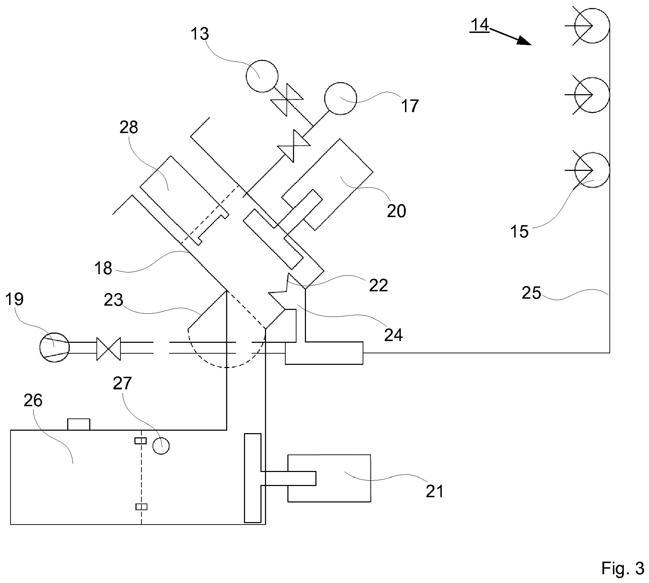

Figur 3 zeigt den schematischen Aufbau des Leitungssystems. Der Benutzer wird einen schematisch gezeigten Behälter 28 in eine Behälteraufnahme 18 einlegen. Der Behälter 28 ist, anders als hier gezeigt, ein länglicher, polyedrischer Körper, welcher mit der Behälteraufnahme 18 formschlüssig zusammenwirkt. Zudem enthält der Behälter 28 einen RFID-Chip, der über die Behälteraufnahme 18 ausgelesen und mit einer Datenbank abgeglichen wird. Erfolgt aufgrund des Formschlusses mit einer Behältermatritze 22 und der Datenabfrage eine Authentifizierung, so wird der Behälter 28 über den Ansaugstutzen 24 geöffnet und in einen nicht gezeigten Zwischenspeicher ausgesaugt.

Der erforderliche Saugdruck kommt von einem Druckaggregat 19. Dieses sorgt auch dafür, dass das Sonnenschutzmittel aus dem Behälter 28 über die Druckleitung 25 zu den Düsenelementen 15 der Düsenanordnung 14 geleitet wird und dort ausgebracht werden kann.

Nach einer Entleerung des Behälters 28 wird dieser mittels eines ersten Druckzylinders gepresst und über eine Auslassklappe 23 in einen abnehmbaren Abfallbehälter 26 verbracht. Dessen Füllstand ist über einen Füllstandssensor 27 abfragbar, bedarfsweise kann der Inhalt des Abfallbehälters 26 über einen zweiten Druckzylinder 21 verdichtet werden. Die Behälteraufnahme 18 kann zudem über einen Wasserzulauf 17 und einen Reinigungsmittelzulauf 13 durchgespült werden.

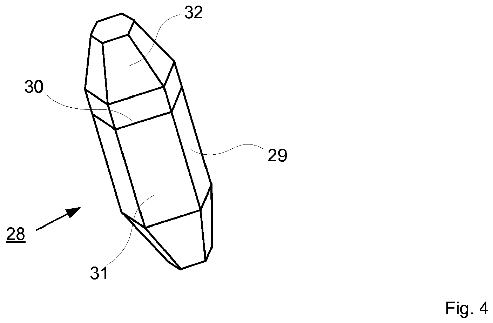

Figur 4 zeigt schließlich eine mögliche Ausgestaltung des Behälters 28. Dieser besteht aus einem Behältermantel 29, der über eine Trennkante 30 in eine erste Teilkammer 31 und eine zweite Teilkammer 32 getrennt werden kann. Beide Teilkammern 31 und 32 sind jeweils flüssigkeitsdicht abgeschlossen. Während die erste Teilkammer nach dem Trennen in die Behälteraufnahme 18 eingelegt wird, verbleibt die zweite Teilkammer bei dem Benutzer, der sie leicht öffnen und die von dem Duschvorgang unberücksichtigten Körperstellen damit einreiben kann.

Vorstehend beschrieben ist somit eine Vorrichtung zum Auftragen von Sonnenschutzmitteln, welche den Grad der Individualisierung im Gegensatz zum Stand der Technik erheblich verbessert. Dies gelingt, indem eine Duscheinheit mit einer Behälteraufnahme vorgesehen wird, in die ein individueller Behälter eingesetzt werden kann, deren Inhalt dann im Inneren der Duscheinheit versprüht wird.

### BEZUGSZEICHENLISTE

- 1: Duscheinheit
- 2: Innenraum
- 3: Umfangswandung
- 4: Lichtschranke
- 5: Mittel zur Bestimmung der Körpergröße
- 6: Fluiddüsenanordnung
- 7: Abfluss
- 8: Außenraum
- 9: Bodenklappe
- 10: Fettabscheider
- 11: Reinigungsmittelbehälter
- 12: Reinigungsdüsenanordnung
- 13: Reinigungsmittelzulauf
- 14: Düsenanordnung
- 15: Düsenelement
- 16: Schiene
- 17: Wasserzulauf
- 18: Behälteraufnahme
- 19: Druckaggregat
- 20: erster Druckzylinder
- 21: zweiter Druckzylinder
- 22: Behältermatritze
- 23: Auslassklappe
- 24: Ansaugstutzen
- 25: Druckleitung
- 26: Abfallbehälter
- 27: Füllstandssensor
- 28: Behälter
- 29: Behältermantel
- 30: Trennkante
- 31: erste Teilkammer
- 32: zweite Teilkammer

## Patentansprüche

1. Vorrichtung zum Auftragen von Sonnenschutzmitteln auf die menschliche Haut, umfassend eine Umfangswandung (3) mit einer Düsenanordnung (14) zum Ausbringen des Sonnenschutzmittels in einen von der Umfangswandung (3) umgebenen Raumbereich, wobei die Düsenanordnung (14) über eine Druckleitung (25) mit einer Behälteraufnahme (18) verbunden ist,
**dadurch gekennzeichnet, dass** die Behälteraufnahme (18) Mittel zur Behälterauthentifizierung sowie Mittel zur Behälteröffnung aufweist, wobei den Mitteln zur Behälteröffnung ein Ansaugstutzen (24) und ein mit diesem verbundenes Druckaggregat (19) zum Ansaugen von Sonnenschutzmittel aus einem in die Behälteraufnahme (18) eingesetzten Behälter (28) in die Druckleitung (25) und zum Ausbringen von Sonnenschutzmittel aus der Druckleitung (25) über die Düsenanordnung (14) in den von der Umfangswandung (3) umgebenen Raumbereich zugeordnet ist.

2. Vorrichtung gemäß Anspruch 1, **dadurch gekennzeichnet, dass** es sich bei den Mitteln zur Behälterauthentifizierung um eine polyedrisch geformte Behältermatritze (22) zur formschlüssigen Aufnahme des Behälters (28) und/oder um eine Leseeinrichtung für optische und/oder elektronische und/oder magnetische Kennzeichnungsmittel handelt.

3. Vorrichtung gemäß einem der vorhergehenden Ansprüche 1 oder 2,
**dadurch gekennzeichnet, dass** die Behälteraufnahme (18) innerhalb eines, den von der Umfangswandung (3) umgebenen Raumbereich umschließenden, Außenraum (8) angeordnet ist.

4. Vorrichtung gemäß einem der vorhergehenden Ansprüche, **dadurch gekennzeichnet, dass** der Druckleitung (25) ein Zwischenspeicher zur Aufnahme wenigstens einer vollständigen Behälterfüllung an Sonnenschutzmittel zugeordnet ist.

5. Vorrichtung gemäß Anspruch 4, **dadurch gekennzeichnet, dass** der Druckleitung (25) ein zuschaltbarer Reinigungsmittelbehälter (11) und/oder ein zuschaltbarer Wasseranschluss zur Verbindung der Druckleitung (25) mit einer Wasserversorgung zugeordnet ist.

6. Vorrichtung gemäß Anspruch 5, **dadurch gekennzeichnet, dass** der Druckleitung (25) eine von der Düsenanordnung (14) separate, insbesondere zusätzliche, Reinigungsdüsenanordnung (12) zur Benetzung der Umfangswandung (3) mit Reinigungsmittel aus dem Reinigungsmittelbehälter (11) zugeordnet ist.

7. Vorrichtung gemäß einem der vorhergehenden Ansprüche, **dadurch gekennzeichnet, dass** der Umfangswandung (3) Mittel zur Bestimmung der Körpergröße (5) einer innerhalb der Umfangswandung (3) stehenden Person zugeordnet sind.

8. Vorrichtung gemäß Anspruch 7, **dadurch gekennzeichnet, dass** es sich bei den Mitteln zur Bestimmung der Körpergröße (3) um wenigstens eine Lichtquelle und mehrere in unterschiedlichen Höhenlagen diese umgebend oder ihr gegenüberliegend angeordnete Fotorezeptoren handelt.

9. Vorrichtung gemäß einem der Ansprüche 7 oder 8, **dadurch gekennzeichnet, dass** die Düsenanordnung (14) aus mehreren Düsenelementen (15) gebildet ist, welche jeweils mit der Druckleitung (25) verbunden sind und in Abhängigkeit von einer ermittelten Körpergröße entlang wenigstens einer vertikalen Schiene (16) entlang der Umfangswandung (3) höhenverstellbar sind.

10. Vorrichtung gemäß einem der vorhergehenden Ansprüche, **dadurch gekennzeichnet, dass** der Umfangswandung (3) eine, vorzugsweise höhenverstellbare, Fluiddüsenanordnung (6) zum Ausblasen eines abwärts gerichteten, den von der Umfangswandung (3) umgebenen Raumbereich umschließenden Fluidmantel zugeordnet ist.

11. Behälter zur Verwendung mit einer Vorrichtung gemäß einem der vorhergehenden Ansprüche, umfassend einen Behältermantel (29) und eine von dem Behältermantel (29) umschlossene, mit Sonnenschutzmittel gefüllte Kammer, **dadurch gekennzeichnet, dass** der Behältermantel (29) eine, vorzugsweise langgestreckte, polyedrische Außenkontur aufweist.

12. Behälter gemäß Anspruch 11, **dadurch gekennzeichnet, dass** die Kammer in eine erste Teilkammer (31) und eine zweite Teilkammer (32) unterteilt ist, wobei die beiden Teilkammern (31, 32) voneinander flüssigkeitsdicht getrennt sind und der Behältermantel (29) über einen Verschluss oder eine Trennkante (30) in zwei Behältermantelteile mit jeweils einer der beiden Teilkammern (31, 32) zerteilbar ist.

13. Behälter gemäß einem der Ansprüche 11 oder 12, **dadurch gekennzeichnet, dass** der Behältermantel (29) aus einem biologisch abbaubaren Kunststoff hergestellt ist.
